(19) Europäisches Patentamt
European Patent Office
Office européen des brevets

(11) Publication number: 0 468 703 A1

# (12) EUROPEAN PATENT APPLICATION

(21) Application number: 91306544.7

(51) Int. Cl.[5]: A61K 7/075

(22) Date of filing: 18.07.91

(30) Priority: 23.07.90 GB 9016099

(43) Date of publication of application:
29.01.92 Bulletin 92/05

(84) Designated Contracting States:
AT BE CH DE DK ES FR GB GR IT LI NL SE

(71) Applicant: UNILEVER PLC
Unilever House Blackfriars P.O. Box 68
London EC4P 4BQ (GB)
(84) GB

(71) Applicant: UNILEVER NV
Burgemeester s'Jacobplein 1 P.O. Box 760
NL-3000 DK Rotterdam (NL)
(84) BE CH DE DK ES FR GR IT LI NL SE AT

(72) Inventor: Cordery, Caroline Susan
11A Warburton Drive, Hale Barns
Altrincham, Cheshire WA5 0SL (GB)
Inventor: Stanley, Susan Jane
53 Mill Lane
Upton by Chester, Cheshire CH2 1BS (GB)
Inventor: McGee, Thomas
55 Voie des Bans, BP 24
F-95102 Argenteuil Cedex (FR)

(74) Representative: Tonge, Robin James et al
UNILEVER PLC Patents Division P.O. Box 68
Unilever House
London EC4P 4BQ (GB)

## (54) Shampoo system.

(57) A shampoo system comprises:
(A) a first pack containing a base shampoo composition comprising a surfactant chosen from anionic, nonionic and amphoteric surfactants, and mixtures thereof, and a cationic conditioning polymer; and
(B) a second pack containing a shampoo additive composition comprising particles of a benefit agent which imparts a benefit to the hair, the particles having an average size of less than 2μm, wherein the first and second packs are adapted to be mixed together before use.

EP 0 468 703 A1

## BACKGROUND OF THE INVENTION

The present invention relates to shampoo systems, and more particularly to shampoo systems comprising a first pack containing a base shampoo composition, and a second pack containing a shampoo additive composition. The first and second compositions are adapted to be mixed together before use in order to impart a particular desired effect to the hair.

The condition and appearance of the hair is influenced by many factors, including environmental conditions, cyclical changes in the body or the effects of stress. Hair products in general are designed to meet a particular need, for example anti-dandruff shampoos, or shampoos for dry or for greasy hair, and a single hair product will at times, not meet the precise requirements of the user.

Additionally shampoos used on different occasions may be required to provide different effects. For example, a user may require that a shampoo imparts an exotic fragrance to the hair for evening, but for daytime the same user may prefer a light fragrance.

Therefore a shampoo system which can provide consumers with a shampoo designed to match their moods, particular occasions or the state of their hair would be particularly advantageous.

Accordingly the invention provides a shampoo system comprising:

(A) a first pack containing a base shampoo composition comprising a surfactant chosen from anionic, nonionic and amphoteric surfactants, and mixtures thereof, and a cationic conditioning polymer which is a cationic derivative of guar gum; and

(B) a second pack containing a shampoo additive composition comprising particles of a benefit agent which imparts a benefit to the hair, the particles having an average size of less than 2μm, wherein the first and second packs are adapted to be mixed together before use.

## DETAILED DESCRIPTION OF THE INVENTION

### Base composition

The shampoo system of the invention comprises a first pack containing a shampoo base composition which comprises a surfactant chosen from anionic, nonionic and amphoteric surfactants, and mixtures thereof.

Suitable anionic surfactants are the alkyl sulphates, alkyl ether sulphates, alkaryl sulphonates, alkyl succinates, alkyl sulphosuccinates, N-alkoyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl groups generally contain from 8 to 18 carbon atoms and may be unsaturated. The alkyl ether sulphates, alkyl ether phosphates and alkyl ether carboxylates may contain from one to 10 ethylene oxide or propylene oxide units per molecule, and preferably contain 2 to 3 ethylene oxide units per molecule.

Examples of suitable anionic surfactants include sodium oleyl succinate, ammonium lauryl sulphosuccinate, ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, triethanolamine lauryl sulphate, triethanolamine monolauryl phosphate, sodium lauryl ether sulphate 1EO, 2EO and 3EO, ammonium lauryl sulphate and ammonium lauryl ether sulphate 1EO, 2EO and 3EO.

The nonionic surfactants suitable for use in the composition of the invention may include condensation products of aliphatic ($C_8$-$C_{18}$) primary or secondary linear or branched chain alcohols or phenols with alkylene oxides, usually ethylene oxide and generally having from 6 to 30 ethylene oxide groups.

Other suitable nonionics include mono- or di-alkyl alkanolamides or alkyl polyglucosides. Examples include coco mono- or di-ethanolamide, coco mono-isopropanolamide, and coco di-glucoside.

The amphoteric surfactants suitable for use in the composition of the invention may include alkyl amine oxides, alkyl betaines, alkyl amidopropyl betaines, alkyl sulphobetaines, alkyl glycinates, alkyl carboxyglycinates, alkyl amphopropionates, alkyl amidopropyl hydroxysultaines, acyl taurates and acyl glutamates wherein the alkyl and acyl groups have from 8 to 18 carbon atoms. Examples include lauryl amine oxide, cocodimethyl sulphopropyl betaine and preferably lauryl betaine, cocamidopropyl betaine and sodium cocamphopropionate.

The surfactants are preferably present in the base shampoo composition of the invention in an amount of from 2 to 40% by weight, and most preferably from 5 to 30%, based on the weight of the base composition.

The base shampoo composition also comprises a cationic conditioning polymer which is a cationic derivative of guar gum.

The deposition agent is preferably a cationic derivative of guar gum.

We have found that in the final shampoo system, when the base and the additive packs are mixed together, that the combination of the cationic conditioning polymer and the benefit agent having an average particle size

of less than 2μm, gives a surprising effect. The particles of benefit agent are found to more effectively deposit on the hair, imparting the desired benefit.

Suitable cationic guar gum derivatives are those prepared by the reaction of the hydroxyl groups of the polygalactomannam gum and reactive quaternary ammonium compounds. The degree of substitution of the cationic groups is preferably in the range of from 0.01 to 0.5. Suitable guar gum derivatives those given the CTFA designated guar hydroxypropyltrimonium chloride, available commercially as JAGUAR for example, JAGUAR C-13-S, in which the degree of substitution is about 0.13. Another suitable material is JAGUAR C-17 which is similar to JAGUAR C-13-S but has a higher degree of substitution of cationic groups of about 0.25 to 1.31. A further example is the hydroxypropylated cationic guar derivative, JAGUAR C-16 which as well as containing the above cationic quaternary groups, also contains hydroxypropyl substituent groups. In JAGUAR C-16 the degree of substitution of the cationic groups is from 0.11 to 0.16, and the moles of substitution of hydroxypropyl groups is from 0.8 to 1.1.

The cationic conditioning polymer is preferably present in the base composition in an amount of from 0.01 to 2% most preferably from 0.04 to 1%, based on the weight of the base composition.

The base shampoo composition according to the invention may also further comprise ingredients conventionally found in shampoo compositions. Examples of such ingredients include foam boosters, thickeners, opacifiers, colouring agents, preservatives, protein derivatives or pH adjustors.

The pH of the base composition is preferably in the range of from 3 to 9, most preferably from 4.5 to 7.5.

## SHAMPOO ADDITIVE COMPOSITION

The shampoo system of the invention also comprises a second pack containing a shampoo additive composition comprising particles of a benefit agent which imparts a benefit to the hair, the particles having an average size of less than 2μm, preferably from 0.01 to 1μm. The average particle size may be measured for example by a laser light scattering technique using a 2600D Particle Sizer from Malvern Instruments.

The benefit agent is present in the shampoo additive composition as particle, these particles may be emulsified droplets of benefit agent, or may be particles of benefit agent dispersed in water.

The benefit agent is preferably chosen from sunscreens, insoluble, non-volatile silicones, silicone resins, perfumes, hair growth agents, hair moisturisers, anti-dandruff agents, bodying agents, shine enhancers or setting agents.

Examples of sunscreens include oil sunscreens such as 2-ethylhexyl-p-methoxy cinnamate, sold commercially as Parsol MCX, 2-ethoxy ethyl-p-methoxy cinnamate, sold commercially as Giv-Tan F, isoamyl-p-methoxy cinnamate, sold commercially as Neo-Heliopan E1000 and 3-(4-methyl benzylidene) camphor sold commercially as Eusolex 6300. Also suitable are oil-soluble sunscreens for example benzophenone derivatives such as 4,4'-tetrahydroxy-benzophenone, sold commercially as Uvinul D50, and 2-hydroxy-4-methoxy-benzophenone, sold commercially as Eusolex 4360,dibenzoyl methane derivatives such as t-butyl-4-methoxydibenzoyl methane, sold commercially as Parsol 1789, and isopropyldibenzoyl methane, sold commercially as Eusolex 8020.

When the sunscreen material comprises oil-soluble sunscreen it is preferably predissolved in an oil before being emulsified. This oil may be an oil sunscreen, or another oil such as 1-octanol, isopropyl myristate or benzyl benzoate.

Examples of shine enhancers are perfluoropolyethers which include those sold commercially as Fomblin HC or Demnam.

Suitable insoluble, non-volatile silicones include polyalkyl siloxanes, polyalkylaryl siloxanes or mixtures thereof.

Suitable non-volatile polyalkyl siloxanes include polydimethyl siloxanes having a viscosity of from 10 to 300,000 centistokes at 25°C. These siloxanes are available commercially from the General Electric Company as the Viscasil series and from Dow Corning as 200 fluids.

The viscosity can be measured by means of a glass capillary viscometer as set forth in Dow Corning Corporate Test Method CTM004 July 20, 1970.

Also suitable is polydiethyl siloxane.

Examples of suitable polyalkylaryl siloxanes include polymethylphenyl polysiloxanes having a viscosity of from 15 to 65 centistokes at 25°C. These siloxanes are available commercially from the General Electric Company as SF 1075 methyl phenyl fluid or from Dow Corning as 556 Cosmetic Grade Fluid.

Aminofunctional silicones such as Dow Corning's DC 929 may also be included in the shampoo additive composition according to an aspect of the invention.

Also suitable are siloxane gums, such as those described in US 4 152 416 (Spitzer), and on General Electric Silicone Rubber product Data Sheet SE 30 , SE 33, SE 54 and SE 76. "Siloxane gum" denotes polydiorganosiloxanes having a molecular weight of from 200,000 to 1,000,000, and specific examples include polydimethyl siloxane copolymer, polydimethyl siloxane/diphenyl/methylvinylsiloxane copolymer, polydimethylsiloxane/methylvinylsiloxane copolymer and mixtures thereof.

Silicone resin material which may be used in the preparation of the shampoo additive composition include oligomerous alkylpolysiloxanes, arylpolysiloxanes or alkylarylpolysiloxanes, composed of suitable combinations of $R_3SiO_{0.5}$ units, $R_2SiO$ units, $RSiO_{1.5}$ units and $SiO_2$ units. Their ratio is selected so that the resin has average formula $R_nSiO_{[(4\ n)/2]}$ where R is $C_{1-6}$ alkyl or aryl and n is from 0.7 to 1.8.

In order to incorporate silicone resins and siloxane gum into the shampoo additive composition, the resin or gum must be predissolved in a volatile solvent, before emulsifying the solution with water to produce an emulsion comprising particles having an average size of less than 2μm.

Suitable resins form films where the volatile solvent is removed by evaporation. These films are hard and brittle at ambient temperature but soften upon being heated. For suitable resins the temperature at which softening begins is preferably greater than 30°C.

It is preferred that the silicone resin has an average molecular weight of from 500 to 10000.

Preferred volatile solvents are those having a boiling point of from 99°C to about 260°C and have a solubility in water of less than about 0.1% solvents are preferably chosen from volatile silicone or volatile hydrocarbon.

The most preferred solvents are volatile silicones, which may be either cyclic or linear polydimethyl siloxanes. Suitable volatile siloxanes are available commercially from Dow Corning as 244,245, 344,345 and 200/5 fluids. Other examples include Silicones 7202 and 7518 from Union Carbide and SWS-03314 from Stauffer Chemical. The number of silicone atoms in the cyclic silicones is 3 to 7, most preferably 4 or 5. The general formula for cyclic silicones is:

CH3

[Si - O]n

CH3

wherein n = 3-7. Viscosities are generally less than 10 centipoise (cP) at 25°C.

Linear polydimethyl siloxanes useful as solvents generally have viscosities of about less than about 5cP at 25°C. The linear volatile silicones contain from about 3 to about 9 silicon atoms and have the general formula

$$(CH_3)_3\ Si\text{-}O\text{-}[Si\ (CH_3)_2O]_nSi\ (CH_3)_3$$

wherein n = 1-7.

Silicones of the above described types are widely available eg from Dow Corning as 244, 245, 344, 345, 200/5 and 200 fluids; Union Carbide as Silicone 7202 and 7158, and Stauffer Chemical as SWS-03314.

When the solvents are hydrocarbons, they may be for example, straight chain or branched chain alkyl and contain from 10 to 16 carbon atoms, preferably from 12 to 16 carbon atoms. Suitable examples are n-dodecane, permethylhydrocarbons 99A and 101A available from Presperse Inc.

Perfumes, deoperfumes, hair growth agents, nutrient agents such as dioleyl glycerol and hair moisturisers such as gamma linolenic acid may also be used as the benefit agent in the shampoo additive composition.

Other suitable benefit agents include anti-dandruff agents such as zinc pyrithione, selenium disulphide, 1-hydroxy-2-pyridone, 1-chlorophenoxy,1-imidazolyl-2-butanone or derivatives thereof. Preferred anti-dandruff agents are 1-hydroxy-4-methyl-6-(2,4,4-trimethyl pentyl)-2-pyridone monoethanolamine salt sold under the trade name OCTOPIROX by Hoechst AG, and 1-chlorophenoxyl-1-(1-imidazolyl)-3', 3-dimethyl-2-butanone, available commercially from Bayer under the trade name CLIMBAZOLE.

Bodying agents such as chitosan and its derivatives, and setting agents such as latex polymers may also be included as benefit agents.

The additive composition is preferably an aqueous emulsion of a benefit agent or a combination of benefit agents in water. The emulsion of the agent or mixture of agents in water can be prepared by mechanical mixing or emulsifying the agent(s) with water and an emulsifier, or by a combination of mechanical and chemical emulsification, to produce an emulsion which comprises particles or "oil" droplets having an average diameter of less than 2μm, preferably from 0.01 to 1μm.

The particles or droplets may be emulsions of the benefit agent(s) alone, when the benefit agent(s) is water insoluble e.g. oil-soluble sunscreens such as Parsol MCX, insoluble nonvolatile silicones such as dimethicones

having a viscosity from 10 to 300000 centistokes at 25°C e.g. DC200 series, or other oil-soluble materials.

Alternatively the particles or droplets may be formed by predissolving the benefit agent in an oily material which is then emulsified.

Any surfactant material either alone or in admixture may be used as the emulsifier in the preparation of the shampoo additive emulsions. Preferred emulsifiers include anionic emulsifiers such as alkylarylsulphonates e.g. sodium dodecylbenzene sulphonate, alkyl sulphates e.g. sodium lauryl sulphate, alkyl ether sulphates e.g. sodium lauryl ether sulphate nEO, where n is from 1 to 20, alkylphenol ether sulphates e.g. octylphenol ether sulphate nEO where n is from 1 to 20, and sulphosuccinates e.g. sodium dioctylsulphosuccinate.

Also suitable are nonionic emulsifiers such as alkylphenol ethoxylates e.g. nonylphenol ethoxylate nEO, where n is from 1 to 50, alcohol ethoxylates e.g. lauryl alcohol nEO, where n is from 1 to 50, ester ethoxylates e.g. polyoxyethylene monostearate where the number of oxyethylene units is from 1 to 30.

The shampoo additive composition according to the invention may also further comprise other ingredients such as preservatives, colouring materials, perfume, suspending agents, pearlisers or pH control agents.

The benefit agent may also be present in the shampoo additive composition as a dispersion in water. The average particle size of the benefit agent must be less than 2μm. The dispersion is preferably stabilised by a suspending polymer eg. polyacrylic acid, cross-linked polymers of acrylic acid, copolymers of acrylic acid with a hydrophobic monomer, copolymers of carboxylic acid-containing monomers and acrylic esters, cross-linked copolymers of acrylic acid acrylate esters, and heteropolysaccharide gums.

Polyacrylic acid is available commercially as Carbopol 420, Carbopol 488 or Carbopol 493 (Goodrich). Polymers of acrylic acid cross-linked by a polyfunctional agent which are suitable for use in the shampoo compositions of the invention include those available commercially as Carbopol 910, Carbopol 934, Carbopol 940, and Carbopol 941 (Goodrich).

An example of a suitable copolymer of a carboxylic acid containing monomer and acrylic esters is Carbopol 1342 (Goodrich). Suitable cross-linked polymers of acrylic acid and acylate esters are Pemulen TR1 or Pemulan TR2. A suitable heteropolysaccharide gum is xantham gum, for example that available as Kelzan mu.

Use of the composition

The first and second packs are adapted to be mixed together before use. The two packs are preferably mixed together in a ratio of base to additive of from 99:1 to 1:99, most preferably of from 90:10 to 50:50.

Mixing may be achieved by shaking, stirring or by mechanical methods of mixing such as shear stirring.

The shampoo system of the invention is applied in an amount of from 3 to 5ml to wet hair. The wet hair is worked to create a lather. The lather may be retained on the head for a short time before rinsing eg. from 1 to 4 minutes, or may immediately be rinsed. The washing procedure may be repeated if required.

## EXAMPLES

The invention is further illustrated by the following examples, in which base shampoo compositions and shampoo additive compositions are respectively intended for filling first and second packs according to the invention.

Example 1

A base shampoo composition having the following composition is prepared:

| | | %wt |
|---|---|---|
| Sodium lauryl ether sulphate 2EO | | 16.0 |
| Cocamidopropyl betaine | | 2.0 |
| Ethylene glycol distearate | | 1.5 |
| JAGUAR C-13-S | | 0.1 |
| Perfume, colouring, preservative | | qs |
| Water | to | 100 |

Example 2

A base shampoo composition having the following composition is prepared:

| | | %wt |
|---|---|---|
| Ammonium lauryl sulphate | | 12.0 |
| Lauryl betaine | | 4.0 |
| JAGUAR C-13-S | | 0.5 |
| Perfume, colouring, preservative | | qs |
| Water | to | 100 |

Example 3

A shampoo additive composition comprising the following ingredients was prepared:

| | | %wt |
|---|---|---|
| DC 200 (60000) | | 2.0 |
| Carbopol 940 | | 0.4 |
| Perfume, colouring, preservative | | qs |
| Water | to | 100 |

This additive composition confers conditioning benefits to the hair when it is used as part of the shampoo system of the invention.

Example 4

A shampoo additive composition comprising the following ingredients was prepared:

| | | %wt |
|---|---|---|
| Zinc pyrithione | | 0.5 |
| Carbopol 940 | | 0.4 |
| Perfume, colouring, preservative | | qs |
| Water | to | 100 |

This additive composition imparts an anti-dandruff benefit to the hair when it is used as part of the shampoo system of the invention.

Example 5

A shampoo additive emulsion comprising the following ingredients was prepared:

| | | %wt |
|---|---|---|
| Polydimethylsiloxane (60,000cSt) | | 50.0 |
| Lauryl alcohol ethoxylate 2EO | | 2.0 |
| Lauryl alcohol ethoxylate 21EO | | 2.0 |
| Preservative | | qs |
| Water | to | 100 |

This additive emulsion improves the softness and manageability of hair when it is used as part of the shampoo system of the invention.

Example 6

A shampoo additive emulsion comprising the following ingredients was prepared:

| | %wt |
|---|---|
| Parsol MCX | 20.0 |
| Xanthan gum | 1.0 |
| Perfume, colouring, preservative | qs |
| Water | to 100 |

This additive composition imparts a sunscreen benefit to the hair when it is used as part of the shampoo system of the invention.

Example 7

A shampoo additive composition having the following composition was prepared::

| | %wt |
|---|---|
| Sodium lauryl ether sulphate 2EO | 16.0 |
| Cocamidopropyl betaine | 2.0 |
| Carbopol 940 | 0.4 |
| Dimethicone (60,000 cSt) | 1.0 |
| Timiron MP1001 | 0.2 |
| Perfume, colouring, preservative | qs |
| Water | to 100 |

Example 8

A shampoo additive composition having the following composition was prepared:

| | %wt |
|---|---|
| Sodium lauryl ether sulphate 2EO | 16.0 |
| Cocamidopropyl betaine | 2.0 |
| Carbopol 940 | 0.4 |
| Perfluoropolyether | 0.0003 |
| Perfume, colouring, preservative | qs |
| Water | to 100 |

Example 9

A shampoo additive composition having the following composition was prepared:

| | %wt |
|---|---|
| Ammonium lauryl sulphate | 12.0 |
| Lauryl betaine | 4.0 |
| Octopirox | 0.75 |
| Perfume, colouring, preservative | qs |
| Water | to 100 |

Example 10

A shampoo additive composition having the following composition was prepared:

| | %wt |
|---|---|
| Sodium lauryl ether sulphate 2EO | 14.7 |
| Tegobetaine L7 | 13.3 |
| Parsol MCX | 1.0 |
| Perfume, colouring, preservative | qs |
| Water | to 100 |

Example 11

A shampoo additive composition having the following was prepared:

| | %wt |
|---|---|
| Sodium lauryl ether sulphate 2EO | 14.7 |
| Tegobetaine L7 | 13.3 |
| Ultrafine titanium dioxide | 2.0 |
| Perfume, colouring, preservative | qs |
| Water | to 100 |

Example 12

A shampoo additive composition having the following composition was prepared:

| | %wt |
|---|---|
| Sodium lauryl ether sulphate 2EO | 14.7 |
| Tegobetaine L7 | 13.3 |
| Ultrafine titanium dioxide | 2.0 |
| Parsol MCX | 1.0 |
| Perfume, colouring, preservative | qs |
| Water | to 100 |

**Claims**

**1.** A shampoo system comprising

(A) a first pack containing a base shampoo composition comprising a surfactant chosen from anionic, nonionic and amphoteric surfactants, and mixtures thereof, and a cationic conditioning polymer; and
(B) a second pack containing a shampoo additive composition comprising particles of a benefit agent which imparts a benefit to the hair, the particles having an average size of less than 2μm, wherein the first and second packs are adapted to be mixed together before use.

**2.** A shampoo system as claimed in Claim 1 wherein the surfactant present in the base shampoo composition is present in an amount of from 2 to 40% by weight of the base shampoo composition.

**3.** A shampoo system as claimed in Claim 1 or Claim 2 wherein the cationic conditioning polymer in the base shampoo composition is present in an amount of from 0.01 to 2% by weight of the base shampoo composition.

**4.** A shampoo system as claimed in Claim 3 wherein the cationic conditioning polymer is a cationic derivative of guar gum.

**5.** A shampoo system according to Claim 4 wherein the cationic derivative of guar gum is guar hydroxypropyl trimonium chloride.

**6.** A shampoo system as claimed in any one of the preceding claims wherein the average size of the particles of benefit agent lies in the range of from 0.01 to 1μm.

**7.** A shampoo system as claimed in any one of the preceding claims wherein the particles of benefit agent comprise droplets of benefit agent emulsified in water.

**8.** A shampoo system as claimed in any one of the preceding claims wherein the benefit agent is selected from sunscreens, insoluble, non-volatile silicones, silicone resins, perfumes, hair growth agents, hair moisturisers, anti-dandruff agents, bodying agents, shine enhancers or setting agents.

**9.** A shampoo system as claimed in any preceding claim wherein the first pack and second pack are mixed together in a ratio of base shampoo composition to shampoo additive composition of from 99:1 to 1:99.

**10.** A shampoo system as claimed in Claim 9 wherein the said ratio lies in the range of from 90:10 to 50:50.

**11.** Use of a shampoo system as claimed in any one of claims 1 to 10 for washing hair.

European Patent Office

# EUROPEAN SEARCH REPORT

Application Number

EP 91306544.7

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 5) |
| Y | EP - A2 - 0 170 927 (BEECHAM GROUP PLC) * Claims; examples; page 4, lines 7-13; page 7 * -- | 1-11 | A 61 K 7/075 |
| Y | PATENT ABSTRACTS OF JAPAN, unexamined applications, C field, vol. 10, no. 350, 26 November 1986 THE PATENT OFFICE JAPANESE GOVERNMENT page 80 C 387 * Kokai-no. 61-151 111 (KAO CORP.) * -- | 1-11 | |
| Y | US - A - 3 753 916 (J.J. PARRAN) * Column 2, lines 64-68; column 5, line 74 - column 6, line 39; column 7, lines 20-30; column 8, lines 50-54; example 3 * -- | 1-11 | TECHNICAL FIELDS SEARCHED (Int. Cl.5) |
| Y | GB - A - 2 107 586 (BEECHAM GROUP PLC) * Page 1, lines 47-60; examples; claims * -- | 1-11 | A 61 K 7/00 |
| A | US - A - 4 557 928 (D.A. GLOVER) * Claims * -- | 1-11 | |
| A | EP - A2 - 0 285 389 (THE PROCTER & GAMBLE CO.) * Pages 3-5 * ---- | 1-11 | |

The present search report has been drawn up for all claims

| Place of search | Date of completion of the search | Examiner |
|---|---|---|
| VIENNA | 17-10-1991 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)